# EUROPEAN PATENT APPLICATION

(11) **EP 2 671 873 A1**
(43) Date of publication of application: **11.12.2013**
(21) Application number: 12171094.1
(22) Date of filing: 06.06.2012
(51) Int. Cl.: C07D 231/12

(54) **Method for the cyclization of hydrazinoacrylic acid derivatives**

(71) Applicant: SOLVAY SA, 1120 Bruxelles (BE)
(72) Inventor: Jaunzems, Janis, 30559 Hannover (DE)
(74) Representative: Mross, Stefan P.M.

(57) **Abstract**

Compounds of formula (I) 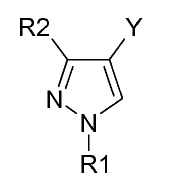 wherein R¹ is an organic substituent, R² is selected from C1 to C4 alkyl groups substituted by one, two or three halogen atoms selected from the group consisting of F, Cl and Br or a CF₃ group, with the proviso that R² is substituted by at least one Cl atom, and Y is selected from the group consisting of C(O)OR³, CN and C(O)NR⁴R⁵ can be produced by UV-light initiated cyclization of compounds of formula (II)

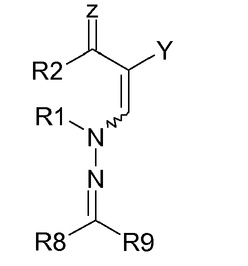

The compounds of formula (I) may be used as intermediates in the manufacture of fungicides.

## Description

The present invention concerns a method for the cyclization of hydrazinoacrylic acid derivatives to form heterocyclic compounds.

1-substituted pyrazoles, e.g. 1-alkylpyrazoles, as is described in US 2011/000962, are intermediates for fungicides.

Object of the present invention is to provide an improved method for the manufacture of heterocycles with an =N-N(R¹) group, e.g. for the manufacture of 3-haloalkylpyrazole-4-carboxylic acid derivates substituted in the C1 position by an R¹ group which is an organic substituent, especially an alkyl group.

Accordingly, the present invention concerns a method for the manufacture of 1-alkyl-3-haloalkyl-pyrazole-4-carboxylic acid derivatives of formula (I)

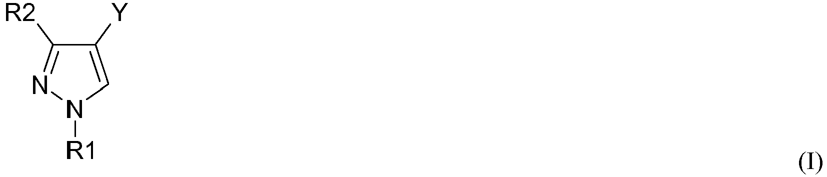

In which R¹ is an organic substituent selected from the group consisting of C1 to C12 alkyl ; C3 to C8 cycloalkyl ; C2 to C12 alkenyl ; C2 to C12 alkynyl ; C6 to C8 aryl ; C7 to C19 arylalkyl ; and C7 to C19 alkylaryl ; each of which may be substituted by one or more groups selected from the group consisting of R', X, OR', SR', NR'₂, SiR'₃, COOR', C(O)R', CN and CONR'₂ wherein R' is H or a C 1 to C12 group ;
R² is selected from C 1 to C4 alkyl groups are substituted by one, two or three halogen atoms selected from the group consisting of F, C1 and Br or a CF₃ group, with the proviso that R² is substituted by at least one C1 atom ; Y is selected from the group consisting of C(O)OR³, CN and C(O)NR⁴R⁵ wherein R³, R⁴ and R⁵ are independently of each other are selected from the group consisting of C1 to C12 alkyl ; C3 to C8 cycloalkyl ; C2 to C12 alkenyl ; C2 to C12 alkynyl ; C6 to C8 aryl ; C7 to C19 arylalkyl ; and C7 to C19 alkylaryl ; each of which may be substituted by one or more groups selected from the group consisting of R', X, OR', SR', NR'₂, SiR'₃, COOR', C(O)R', CN and CONR'₂ wherein R' is H or a C1 to C12 group ; and R⁴ and R⁵ together with the nitrogen atom to which they are attached may form a 5-membered or 6-membered ring which may optionally contain one or more further heteroatoms selected from the group consisting of O, S and an SO₂ group and which may be substituted by one or more groups selected from the group consisting of R', X, OR', SR', NR'₂, SiR'₃, COOR', C(O)R', CN and CONR'₂ wherein R' is H or a C 1 to C12 group ; and X is fluorine, chlorine, bromine and iodine, preferably fluorine or chlorine ;
a) by subjecting a 2-acylated or 2-iminoalkylated acrylic acid hydrazone of the formula (II) 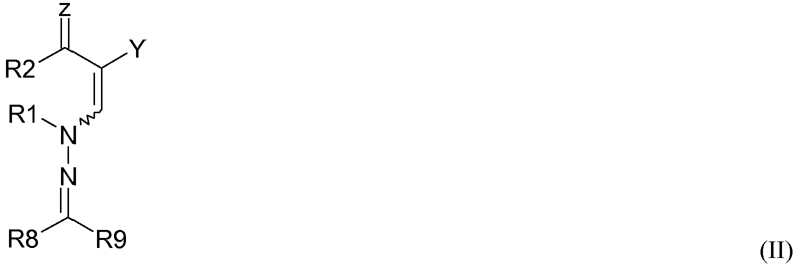 in which R¹ and R² have the meaning given above, Z is selected from the group consisting of O, S and N⁺R⁶R⁷ wherein R⁶ and R⁷ independently from each other are selected from the group consisting of C1 to C12 alkyl ; C3 to C8 cycloalkyl ; C2 to C12 alkenyl ; C2 to C12 alkynyl ; C6 to C8 aryl; C7 to C 19 arylalkyl ; and C7 to C 19 alkylaryl ; each of which may be substituted by one or more groups selected from the group consisting of R', X, OR', SR', NR'₂, SiR'₃, COOR', C(O)R', CN and CONR'₂ wherein R' is H or a C1 to C12 group ;
and R⁶ and R⁷ together with the nitrogen atom to which they are attached may form a 5-membered or 6-membered ring which may optionally contain one or more further heteroatoms selected from the group consisting of O, S and an SO₂ group and which may be substituted by one or more groups selected from the group consisting of R', X, OR', SR', NR'₂, SiR'₃, COOR', C(O)R', CN and CONR'₂ wherein R' is H or a C1 to C12 group ; and X is fluorine, chlorine, bromine and iodine, preferably fluorine or chlorine ;
R⁸ and R⁹ are independently selected from the group consisting of C1
to C12 alkyl ; C3 to C8 cycloalkyl ; C2 to C12 alkenyl ; C2 to C12 alkynyl ; C6 to C8 aryl ; C7 to C19 arylalkyl ; and C7 to C19 alkylaryl ; each of which may be substituted by one or more groups selected from the group consisting of R', X, OR', SR', NR'₂, SiR'₃, COOR', C(O)R', CN and CONR'₂ wherein R' is H or a C1 to C12 group ; and R⁸ and R⁹ together with the nitrogen atom to which they are attached may form a 5-membered or 6-membered ring which may optionally contain one or more further heteroatoms selected from the group consisting of O, S and an SO₂ group and which may be substituted by one or more groups selected from the group consisting of R', X, OR', SR', NR'₂, SiR'₃, COOR', C(O)R', CN and CONR'₂ wherein R' is H or a C1 to C12 group. R⁴, R⁵, R⁶, R⁷, R⁸ and R⁹ are preferably C 1 to C5 alkyl, and more preferably, methyl or ethyl; and X is fluorine, chlorine, bromine and iodine, preferably fluorine or chlorine ; and when Z is N⁺R⁶R⁷ the positive charge is balanced by an anion, e.g. by a sulfate anion or Cl⁻ to UV light to initiate a cyclization reaction.

R¹ preferably is C 1 to C5 alkyl ; or C 1 to C5 alkyl, substituted by at least one halogen atom, and more preferably, R¹ is methyl, ethyl, n-propyl, i-propyl, n-butyl, s-butyl, t-butyl or n-pentyl. Especially preferably, R¹ is methyl or ethyl, most preferably, methyl.

Preferably, R² is chloromethyl, dichloromethyl, trichloromethyl, chlorobromomethyl, chlorofluoromethyl, chlorodifluoromethyl, fluorodichloromethyl, 1-chloroethyl, 2,2-dichloroethyl, 1,2-dichloroethyl, 2-chlorofluoroethyl, 2-chloro-2,2-difluoroethyl, 2,2-dichloro-2-fluoroethyl, 2,2,2-trichloroethyl ; especially preferably, R² is CClF₂. Preferably, Z is O. Preferably, Y is C(O)OR³ wherein R³ is a C1 to C5 alkyl group.

More preferably, R¹ is methyl or ethyl, R² is CHF₂, CClF₂ or CF₃, and R³ is methyl or ethyl.

Especially preferably, R¹ is methyl, R² is CHF₂, CClF₂ or CF₃, and R³ is methyl or ethyl.

R³, R⁴, R⁵, R⁶, R⁷, R⁸ and R⁹ are preferably methyl, ethyl, propyl or butyl, and especially preferably, they are methyl or ethyl.

Preferred pyrazole compounds of formula (I) are assembled in table 1.

**Table 1 : Preferred pyrazole compounds**

| |
|---|
| 3-CF₂Cl-1-CH₃-1 H-pyrazole-4-COOCH₃ |
| 3-CF₂Cl-1-CH₃-1 H-pyrazole-4-COOCH₂CH₃ |
| 3-CF₂Cl-1-CH₃-1 H-pyrazole-4-COOCH₂CH₂CH₃ |
| 3-CFHCl-1-CH₃-1 H-pyrazole-4-COOCH₃ |
| 3-CFHCl-1-CH₃-1 H-pyrazole-4-COOCH₂CH₃ |
| 3-CFHCl-1-CH₃-1 H-pyrazole-4-COOCH₂CH₂CH₃ |
| 3-CH₂Cl-1-CH₃-1 H-pyrazole-4-COOCH₃ |
| 3-CH₂Cl-1-CH₃-1 H-pyrazole-4-COOCH₂CH₃ |
| 3-CH₂Cl-1-CH₃-1 H-pyrazole-4-COOCH₂CH₂CH₃ |

The UV light treatment of the compound of formula (II) preferably is performed in a solvent. Protic, aprotic, polar and nonpolar organic solvents may be used. It is only necessary that the solvent contains water or moisture or a hydrogen atom, e.g. in the form of an O-H group or C-H group. For example, aromatic solvents, e.g. toluene, are suitable. Alcohols are also suitable as solvent, e.g. monobasic alcohols and dibasic alcohols, for example, methanol, ethanol, n-propanol, isopropanol or glycol. Hydrocarbons, be they linear, branched or cyclic, can be applied, for example hydrocarbons with 3 to 10 carbon atoms, e.g. n-butane, 2-methylpropane, any isomers of pentane, e.g. n-pentane, any isomers of hexane, e.g. n-hexane, any isomers of heptane, e,g, n-heptane, and any mixtures thereof. Halohydrocarbons are suitable as solvent, e.g. saturated halohydrocarbons with 1 to 10 carbon atoms wherein halo denotes chlorine and fluorine. For example, dichloromethane, trichloromethane, trichloroethane, any isomers of pentafluoropropane, e.g. 1,1,1,3,3-pentafluoropropane, the two isomers of heptafluoropropane, e.g. 1,1,1,2,3,3,3-heptafluoropropane, any isomers of pentafluorobutane, e.g. 1,1,1,3,3-pentafluorobutane, fluorosubstitutedpentanes, e.g. decafluoropentane, and and any mixtures thereof.

UV light providing irradiation comprising at least one wavelength, or a spectrum of wavelengths, in the range of 190 to 254 nm can be used for the UV treatment of the compound of formula (II). Low pressure mercury lamps and middle pressure UV lamps are very suitable. LEDs and OLEDs providing irradiation having at least one wavelength, or a spectrum of wavelengths, in the UV light region are also suitable. Thus, laser light in the UV is suitable.

It is not necessary that UV light is irradiated into the reaction mixture for the whole duration of the cyclization reaction. It is sufficient to switch it on in the beginning of the reaction to induce the reaction start. For example, it is sufficient to irradiate UV light into the reaction mixture for a time span in the range of 1 second to 5 minutes. If desired, UV light may be irradiated into the reaction mixture intermittently, e.g. every 10 to 30 minutes but this is not necessary.

The advantage of the reaction is, for example, that it must not be performed in a photo reactor. It is only necessary that, to start the reaction, UV light is irradiated shortly into the respective reactor.

The treatment with UV light can be performed in any reactor suitable for UV irradiation of liquid phase components. Stirred batch reactors are very suitable. Flow reactors in which the starting compounds are, usually quickly, passed through lines or other reactor parts in which the starting compounds are contacted with UV light, are also suitable.

The reaction temperature for performing the UV treatment is very variable. For example, it can be performed between -30°C and +100°C. Preferably, the reaction temperature is ranging from-20°C to 30°C.

The pressure is not critical. It is technically especially simple to perform the UV treatment at ambient pressure. But, if desired, the UV treatment can be performed under reduced pressure or at elevated pressure, e.g. at a pressure of equal to or less than 20 bar (abs).

If desired, inert gas, e.g., nitrogen, can be used to prevent the intrusion of air or oxygen into the reactor in which the UV treatment is performed.

The working up of the resulting reaction mixture can be performed according to methods known in the art. For example, solvent can be evaporated off, and the desired compound of formula (I) can be isolated by distillation under a vacuum. The separation of the 3-R² isomer from the 5-R² isomer can be achieved by methods known in the art, especially by distillation in a vacuum. Since the undesired isomer is produced in very low amounts, e.g. of 1 % or less, it is preferred to remove it in a final purification step. For example, it can be removed by crystallization, e.g. in a mixture of 1,1,1,3,3-pentafluorobutane/hexane after a reduction step.

The 1-R¹-3-haloalkyl-pyrazole-4-carboxylic acid derivatives are precursors of fungicidal active compounds ; see for example, US 2011/0009642 and WO 03/070705. Often, in fungicides, the 3-halo group is CHF₂. Thus, if a compound of formula (I) is produced having a CClF₂ group, this group can be reduced to form a difluoromethyl group. The reduction can be performed as described in WO 2012010692 using metal hydrides, H₂/catalyst (e.g. H₂/Pd) or metal/hydrogen source, e.g. Zn/ethanol.

The compounds of formula (II) can be manufactured as described in US 2011/0009462. For example, they can be prepared from 2-acylated or 2-iminoalkylated acrylic acid derivatives and an N-alkylhydrazone (according to step A of US 2011/0009462), by acylation of an acrylic acid hydrazone with an acyl halide (according to step B of US 2011/0009462) or by the aminoalkylation of an acrylic acid hydrazone with an α,α-dihaloamine (according to step C of US 2011/0009462).

Preferably, the compounds of formula (II) are manufactured from compounds of formula (III) and an N-alkylhydrazone of formula (IV) according to the reaction scheme A

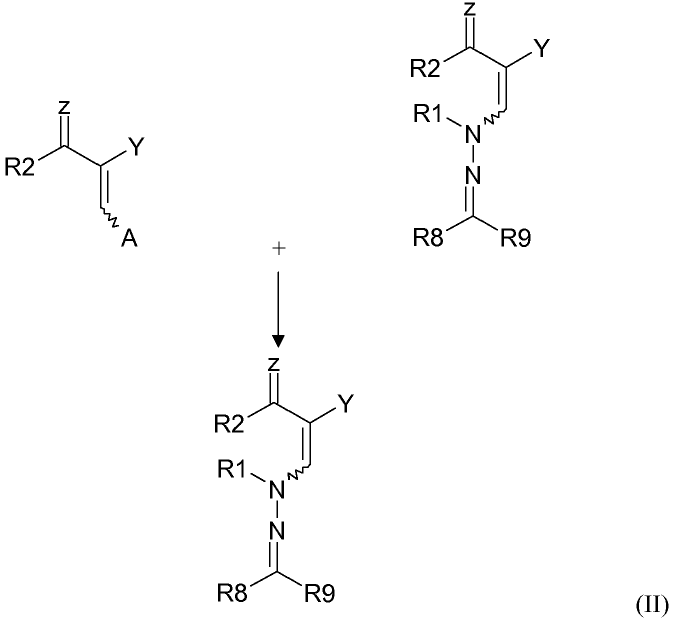

Or, according to the reaction scheme B, from compounds of formula (V) and an N-alkylhydrazone of formula (IV) according to the reaction scheme B

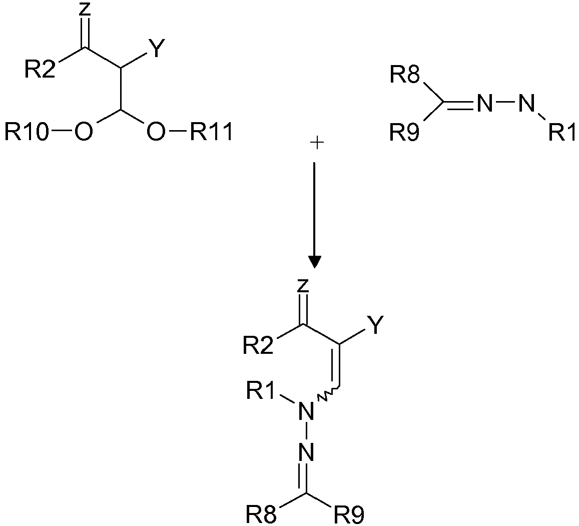

In compounds of formula (III), (IV) and (V), Y, Z, R¹, R², R⁸ and R⁹ have the meanings given above, respectively ; R¹⁰ and R¹¹ independently of each other are C1 to C5 alkyl groups ; and A is a leaving group selected from the group consisting of OR¹², SR¹² and NR¹²R¹³ wherein R¹² and R¹³ independently selected from the group consisting of C1 to C12 alkyl ; C3 to C8 cycloalkyl ; C2 to C12 alkenyl ; C2 to C12 alkynyl ; C6 to C8 aryl ; C7 to C19 arylalkyl ; and C7 to C19 alkylaryl ; each of which may be substituted by one or more groups selected from the group consisting of R', X, OR', SR', NR'₂, SiR'₃, COOR', C(O)R', CN and CONR'₂ wherein R' is H or a C1 to C12 group ; and R¹² and R¹³ together with the nitrogen atom to which they are attached may form a 5-membered or 6-membered ring which may optionally contain one or more further heteroatoms selected from the group consisting of O, S and an SO₂ group and which may be substituted by one or more groups selected from the group consisting of R', X, OR', SR', NR'₂, SiR'₃, COOR', C(O)R', CN and CONR'₂ wherein R' is H or a C 1 to C12 group ; and X is fluorine, chlorine, bromine and iodine, preferably fluorine or chlorine. Preferably, R¹⁰, R¹¹, R¹² and R¹³ are methyl, ethyl, propyl or butyl, and especially, methyl or ethyl.

Compounds of formula (III) are known and can be manufactured according to known methods. The compounds of formula (III) wherein A is OR¹² can be manufactured, e.g. in the presence of acetic acid anhydride, from trialkyl orthoformate of formula (VI), HC(OR¹²)(OR¹⁴)(OR¹⁵), and the respective derivative of 3-oxobutyric acid having the formula (VII), R²-C(O)-CH₂-Y, wherein R² and Y have the meanings and the preferred meanings as given above ; R¹², R¹⁴ and R¹⁵ are the same or different and are C1 to C5 alkyl ; R¹², R¹⁴ and R¹⁵ are preferably methyl, ethyl, propyl or butyl. Especially preferably, they are the same and are methyl, ethyl or n-propyl. Still more preferably, they are the same and are ethyl. The reaction is described in detail in US 2011/0009642. Especially preferably, R² is CClF₂. Preferably, Z is O. Preferably, Y is C(O)OR³ wherein R³ is a C1 to C5 alkyl group.

The preferred compounds of formula (V) wherein Y is C(O)OR³ can be manufactured, in the absence of acetic acid anhydride, by reacting compounds of formula (VIII), R²-C(O)-CH₂-C(O)-OR³, wherein R¹ and R³ have the meaning given above, and trialkylorthoformates having the formula (VI'), HC(OR¹⁰)(OR¹¹)(OR¹²) wherein R¹⁰, R¹¹ and R¹² have the meaning given above. Compounds of formulae (II) and (VIII) can be manufactured by the addition of an acetic acid chloride of formula R²-C(O)Cl to ketene as described in DE-AS 1158490 and subsequent derivatization ; the compounds of formula (VIII) can, for example, be obtained by the reaction of ketene with the acetic acid chloride R²-C(O)Cl and subsequent reaction with an alcohol having the formula R³OH ; R² has the meaning given above.

The respective chemical reaction between compounds of formula (VIII) and formula (VI') is described in unpublished EP patent application N° 12162417.5 filed March 30, 2012, and will be described in detail. It is assumed that the reaction proceeds according to the reaction scheme C

R²-C(O)-CH₂-C(O)-OR³ (VIII) + HC(OR¹⁰)(OR¹¹)(OR¹²) → R²-C(O)-CH(R¹⁶)-C(O)-OR³ (V) + R¹²OH (VI')

To be noted : for simplification, the reaction equation between compounds (VIII) and (VI') given above assumes that R¹⁰, R¹¹ and R¹² are the same. Otherwise, mixtures would be obtained, and different alcohols would be liberated. R¹⁶ is C(H)(OR¹⁰)(OR¹¹).

The reaction according to reaction scheme C between compounds of formula (VIII) and (VI') is performed at a temperature which allows for a reasonably fast reaction. Preferably, the temperature is equal to or greater than 80°C. More preferably, it is equal to or greater than 90°C. Especially preferably, it is equal to or greater than 100°C.

The upper limit of the reaction temperature is selected such that no undesired amounts of side reactions take place. Often, the reaction is performed at a temperature equal to or lower than 180°C, preferably, equal to or lower than 160°C.

If desired, the reaction can be performed in the presence of an inert gas, e.g. it can be performed in the presence of N₂.

If desired, the reaction between compounds of formula (VIII) and (VI') can be performed in the presence of one or more solvents, for example, in the presence of at least one solvent selected from the group consisting of aprotic or protic organic solvents. Those solvents mentioned above as being suitable solvents for the UV treatment may also be used in the reaction according to scheme C. According to a preferred embodiment, an excess of the compound of formula (VI') is applied as solvent. Especially preferably, triethylorthoformate is the preferred compound of formula (VI'), and thus, it is the especially preferred solvent.

The reaction between compounds of formula (VIII) and (VI') is not sensitive to pressure ; it can be performed in a vacuum, at ambient pressure and under pressure above ambient pressure, e.g. at a pressure up to 10 bar (abs) or more. In a preferred embodiment, the resulting alcohol is distilled off, and thus, performing the reaction at ambient pressure or applying a vacuum is preferred. The distilled alcohol is of such a high purity that it can be used for other useful purposes, e.g. as a solvent or as a reagent to produce compounds of formula (VIII) according to the description of DE-AS 1158490.

After completion of the reaction according to scheme C, preferably, any solvent is removed from the reaction mixture. This is preferably performed by applying a vacuum. If, for example, triethyl orthoformate ("TEOF") is applied as starting compound of formula (VI), it is preferred to remove the excess of TEOF by keeping the reaction mixture at approximately 100°C and an initial vacuum of 100 mbar.

Solvent, e.g. trialkyl orthoformate when applied in excess, can be reused.

The resulting compound of formula (V) is obtained in sufficient purity for further reaction steps.

Should the disclosure of any patents, patent applications, and publications which are incorporated herein by reference conflict with the description of the present application to the extent that it may render a term unclear, the present description shall take precedence.

The following examples are intended to explain the invention in further detail without the intention to limit it.

### General remarks :

Triethylorthoformate (TEOF) is commercially available. Ethyl difluorochloroacetoacetate (ECDFAA) is commercially available from Solvay Fluor GmbH, Hannover /Deutschland.

### Example 1 : Manufacture of 4-Chloro-2-diethoxymethyl-4,4-difluoro-3-oxobutyric acid ethyl ester ("ECDFAA-DEM")

TEOF (130 g, 0.88 mol) was placed in a flask equipped with a magnetic stirring bar and a vacuum distillation cooler. ECDFAA (35 g, 0.175 mol) was added.

The resulting mixture was heated to 110°C (outer temperature of the flask) under a mild vacuum (300 mbar) ; ethanol formed during the reaction is slowly distilled out of the vessel. After 2h, the starting material was completely consumed, and product formation was observed by GC analysis. Excess of TEOF was removed from the reaction mixture by distillation (at 100°C, starting with a pressure of 100 mbar at the beginning and 10 mbar at the end, to remove all TEOF traces), resulting pure product as pale yellow liquid with quantitative yield.

¹H NMR (500 MHz, CHLOROFORM-*d*) δ ppm 1.15 (t, 3 H) 1.20 (t, 3 H) 1.27 (t, 3 H) 3.49 - 3.57 (m, 1 H) 3.61 - 3.69 (m, 1 H) 3.71 - 3.79 (m, 2 H) 4.23 (q, 2 H) 4.34 (d, *J*=8.24 Hz, 1 H) 5.18 (d, *J*=8.24 Hz, 1 H). Example 2 : Manufacture of 4-chloro-2-[1-ethoxy-meth-(Z)-ylidene]-4,4-difluoro-3-oxo-butyric acid ethyl Ester ("EME-CDFAA")

Triethylorthoformate (415 g = 2.15 mol), ECDFAA (280 g = 1.4 mol) and acetic acid anhydride (428 g = 4.2 mol) were given into a flask equipped with a vacuum distillation cooler. The resulting mixture was heated to 135°C (outer temperature. Slowly, formed light boilers were distilled off. After 9 hours, the mixture was heated to 110°C under slowly lowering the pressure from ambient pressure to a vacuum of 10 mbar ; all light boilers were distilled out of the reaction mixture. A yellow-brown liquid with a purity of 87 % (determined by gas chromatography) remained in the flask. The raw product could be used immediately without further purification for the next reaction step.

¹H NMR (500 MHz, CHLOROFORM-*d*), δ ppm (the spectrum shows a ratio of E to Z compound of 1:2.5) : 1.25 - 1.35 (4 t, 6 H) ; 4.20 - 4.38 (4 q, 4 H) and 7.76 (bs, 1 H).

### Example 3 : Manufacture of ethyl 4-chloro-4,4-difluoro-2-((1-methyl-2-(propan-2-ylidene)hydrazinyl)methylene)-3-oxobutanoate

### 3.1. Manufacture of N-monomethylhydrazone

To 25 ml of dry acetone, 0.76g (16.5 mmol) of monomethylhydrazine was added. A clear solution resulted. The solution was stirred for 30 min at room temperature.

### 3.2. Manufacture of ethyl 4-chloro-4,4-difluoro-2-((1-methyl-2-(propan-2-ylidene)hydrazinyl)methylene)-3-oxobutanoate

To the clear solution of example 3.1., 5 g (16.5 mmol) of 4-Chloro-2-diethoxymethyl-4,4-difluoro-3-oxo-butyric acid ethyl ester of example 1 was added drop-wise under moderate cooling of the reaction mixture with tap water. The resulting reaction mixture was stirred for 15 h. A sample was taken and analyzed by gas chromatography which revealed a quantitative consumption and formation of ethyl 4-chloro-4,4-difluoro-2-((1-methyl-2-(propan-2-ylidene)hydrazinyl)methylene)-3-oxobutanoate. The crude yellow solution was directly used as starting material in the cyclization reaction of example 4.

### 3.3. Manufacture of ethyl 4-chloro-4,4-difluoro-2-((1-methyl-2-(propan-2-ylidene)hydrazinyl)methylene)-3-oxobutanoate using EME-CDFAA as starting material

Example 3.2. is repeated but EME-CDFAA of example 2 is reacted with t he monomethylhydrazone of example 3.1.

Example 4 : Ethyl 3-(chlorodifluoromethyl)-1-methyl-1H-pyrazole-4-carboxylate

The crude solution of ethyl 4-chloro-4,4-difluoro-2-((1-methyl-2-(propan-2-ylidene)hydrazinyl)methylene)-3-oxobutanoate, resulting from example 3.2, was irradiated in a quartz photo reactor operated with a 20W low pressure Hg lamp. The lamp was switched on for 1 minute and then switched off. The reaction mixture was continued to be stirred for further 10 minutes to complete the cyclizaion reaction. A GC analysis of a sample revealed quantitative conversion of the starting material. Solvent was evaporated yielding pure product in quantitative yield.

Alternative to an isolation, the crude ethyl 3-(chlorodifluoromethyl)-1-methyl-1H-pyrazole-4- carboxylate, dissolved in THF, can be subjected to a reduction reaction with Pd/TiO₂ in the presence of CsF, molecular sieve and K₂CO₃ as described in example 55 in WO 2012/010692.

The product obtained after the hydrodechlorination, ethyl 1-methyl-3-difluoromethyl-pyrazole-4-carboxylate, is an intermediate useful for the manufacture of fungicides.

## Claims

1. A method for the manufacture of 1-alkyl-3-haloalkyl-pyrazole-4-carboxylic acid derivatives of formula (I) 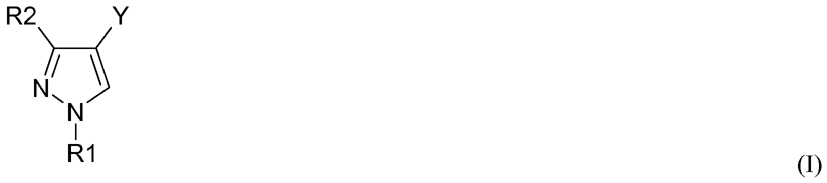 In which R¹ is an organic substituent selected from the group consisting of C1 to C12 alkyl ; C3 to C8 cycloalkyl ; C2 to C12 alkenyl ; C2 to C12 alkynyl ; C6 to C8 aryl ; C7 to C19 arylalkyl ; and C7 to C19 alkylaryl ; each of which may be substituted by one or more groups selected from the group consisting of R', X, OR', SR', NR'₂, SiR'₃, COOR', C(O)R', CN and CONR'₂ wherein R' is H or a C1 to C12 group ;
R² is selected from C1 to C4 alkyl groups are substituted by one, two or three halogen atoms selected from the group consisting of F, C1 and Br or a CF₃ group, with the proviso that R² is substituted by at least one C1 atom ;
Y is selected from the group consisting of C(O)OR³, CN and C(O)NR⁴R⁵ wherein R³, R⁴ and R⁵ are independently of each other are selected from the group consisting of C1 to C12 alkyl ; C3 to C8 cycloalkyl ; C2 to C12 alkenyl ; C2 to C12 alkynyl ; C6 to C8 aryl ; C7 to C19 arylalkyl ; and C7 to C19 alkylaryl ; each of which may be substituted by one or more groups selected from the group consisting of R', X, OR', SR', NR'₂, SiR'₃, COOR', C(O)R', CN and CONR'₂ wherein R' is H or a C1 to C12 group ; and R⁴ and R⁵ together with the nitrogen atom to which they are attached may form a 5-membered or 6-membered ring which may optionally contain one or more further heteroatoms selected from the group consisting of O, S and an SO₂ group and which may be substituted by one or more groups selected from the group consisting of R', X, OR', SR', NR'₂, SiR'₃, COOR', C(O)R', CN and CONR'₂ wherein R' is H or a C1 to C12 group ; and X is fluorine, chlorine, bromine and iodine ;
a) by subjecting a 2-acylated or 2-iminoalkylated acrylic acid hydrazone of formula (II) 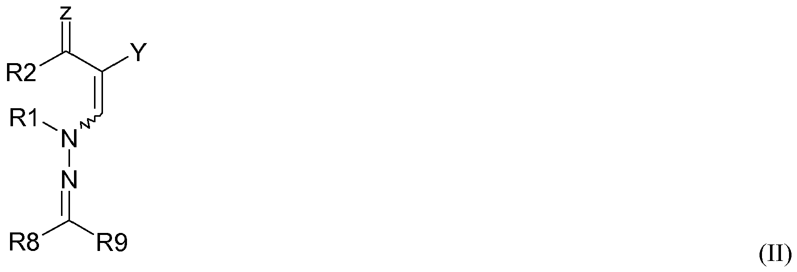
in which R¹ and R² have the meaning given above, Z is selected from the group consisting of O, S and N⁺R⁶R⁷ wherein R⁶ and R⁷ independently from each other are selected from the group consisting of C1 to C12 alkyl ; C3 to C8 cycloalkyl ; C2 to C12 alkenyl ; C2 to C12 alkynyl ; C6 to C8 aryl ; C7 to C 19 arylalkyl ; and C7 to C 19 alkylaryl ; each of which may be substituted by one or more groups selected from the group consisting of R', X, OR', SR', NR'₂, SiR'₃, COOR', C(O)R', CN and CONR'₂ wherein R' is H or a C1 to C12 group ;
and R⁶ and R⁷ together with the nitrogen atom to which they are attached may form a 5-membered or 6-membered ring which may optionally contain one or more further heteroatoms selected from the group consisting of O, S and an SO₂ group and which may be substituted by one or more groups selected from the group consisting of R', X, OR', SR', NR'₂, SiR'₃, COOR', C(O)R', CN and CONR'₂ wherein R' is H or a C1 to C12 group ; and X is fluorine, chlorine, bromine and iodine ;
R⁸ and R⁹ are independently selected from the group consisting of C1 to C12 alkyl ; C3 to C8 cycloalkyl ; C2 to C12 alkenyl ; C2 to C12 alkynyl ; C6 to C8 aryl ; C7 to C19 arylalkyl ; and C7 to C19 alkylaryl ; each of which may be substituted by one or more groups selected from the group consisting of R', X, OR', SR', NR'₂, SiR'₃, COOR', C(O)R', CN and CONR'₂ wherein R' is H or a C1 to C12 group ; and R⁸ and R⁹ together with the nitrogen atom to which they are attached may form a 5-membered or 6-membered ring which may optionally contain one or more further heteroatoms selected from the group consisting of O, S and an SO₂ group and which may be substituted by one or more groups selected from the group consisting of R', X, OR', SR', NR'₂, SiR'₃, COOR', C(O)R', CN and CONR'₂ wherein R' is H or a C1 to C12 group ; and X is fluorine, chlorine, bromine and iodine ; and when Z is N⁺R⁶R⁷ the positive charge is balanced by an anion,
to UV light to initiate a cyclization reaction.

2. The method of claim 1 wherein R¹ is an organic substituent selected from the group consisting of C1 to C5 alkyl.

3. The method of claims 1 or 2 wherein Z is O.

4. The method of anyone of claims 1 to 3 wherein Y is C(O)OR³ wherein R³ is a C1 to C5 alkyl group.

5. The method of anyone of claims 1 to 4 wherein R² is selected from the group consisting of chloromethyl, dichloromethyl, trichloromethyl, chlorobromomethyl, chlorofluoromethyl, chlorodifluoromethyl, fluorodichloromethyl, 1-chloroethyl, 2,2-dichloroethyl, 1,2-dichloroethyl, 2-chlorofluoroethyl, 2-chloro-2,2-difluoroethyl, 2,2-dichloro-2-fluoroethyl, 2,2,2-trichloroethyl.

6. The method of anyone of claims 1 to 5 wherein R² is CClF₂.

7. The method of anyone of claims 1 to 3 wherein R³, R⁴, R⁵, R⁶, R⁷, R⁸ and R⁹ are C1 to C5 alkyl.

8. The method of anyone of claims 1 to 7 wherein the irradiation of UV light is stopped after the initiation of the cyclization reaction.

9. The method of anyone of claims 1 to 8 wherein the UV light is intermittently switched on and off.

10. The method of anyone of claims 1 to 9 wherein ethyl 3-(chlorodifluoromethyl)-1-methyl-1H-pyrazole-4- carboxylate is formed by UV light treatment of ethyl 4-chloro-4,4-difluoro-2-((1-methyl-2-(propan-2-ylidene)hydrazinyl)methylene)-3-oxobutanoate.

11. The method of anyone of claims 1 to 10 wherein the at least one chlorine atom in the R² is substituted by hydrogen.

12. The method of claim 11 wherein the chlorine atom in ethyl 3-(chlorodifluoromethyl)-1-methyl-1H-pyrazole-4- carboxylate is substituted by a hydrogen atom to form ethyl 3-(difluoromethyl)-1-methyl-1H-pyrazole-4-carboxylate.

13. A method for the manufacture of fungicidal pyrazoles comprising a step of manufacturing a compound of formula (I) 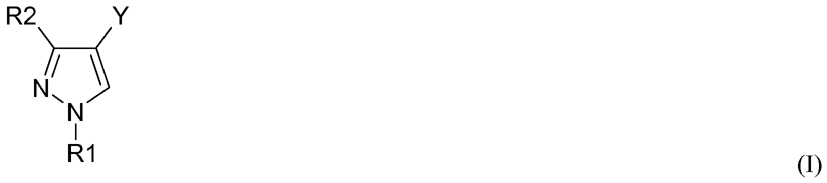 according to anyone of claims 1 to 12, and subjecting the compound of formula (I) to further reaction steps to form a fungicidal compound.
